# EUROPEAN PATENT SPECIFICATION

(11) **EP 2 746 772 B1**
(45) Date of publication and mention of the grant of the patent: **23.03.2016**
(21) Application number: 12198401.7
(22) Date of filing: 20.12.2012
(51) Int. Cl.: G01N 33/543

(54) **Lipid membrane enveloped particles with membrane proteins**
Lipidmembran-umhüllte Partikel mit Membranproteinen
Particules enveloppées de membrane lipidique avec des protéines de membrane

(43) Date of publication of application: 25.06.2014
(73) Proprietor: AIT Austrian Institute of Technology GmbH, 1220 Wien (AT)
(72) Inventor: Naumann, Renate, 1020 Vienna (AT); Nowak, Christoph, 1180 Vienna (AT)
(74) Representative: Sonn & Partner Patentanwälte

(56) References cited:
- GUSTAV NORDLUND ET AL: "A Membrane-Reconstituted Multisubunit Functional Proton Pump on Mesoporous Silica Particles", ACS NANO, vol. 3, no. 9, 22 September 2009 (2009-09-22), pages 2639-2646, XP055054772, ISSN: 1936-0851, DOI: 10.1021/nn9005413
- GIESS F ET AL: "THE PROTEIN-TETHERED LIPID BILAYER: A NOVEL MIMIC OF THE BIOLOGICAL MEMBRANE", BIOPHYSICAL JOURNAL, vol. 87, no. 5, 1 November 2004 (2004-11-01), pages 3213-3220, XP009072540, ISSN: 0006-3495, DOI: 10.1529/BIOPHYSJ.104.046169
- SHARMA M K ET AL: "BACTERIORHODOPSIN CONJUGATES AS ANCHORS FOR SUPPORTED MEMBRANES", BIOCONJUGATE CHEMISTRY, vol. 15, no. 4, 1 July 2004 (2004-07-01), pages 942-947, XP001236872, ISSN: 1043-1802, DOI: 10.1021/BC034231H
- LINA ZHONG ET AL: "Tether-Supported Biomembranes with [alpha]-Helical Peptide-Based Anchoring Constructs", LANGMUIR, vol. 29, no. 1, 8 January 2013 (2013-01-08), pages 299-307, XP055054285, ISSN: 0743-7463, DOI: 10.1021/la303628n
- TROUTIER ET AL: "An overview of lipid membrane supported by colloidal particles", ADVANCES IN COLLOID AND INTERFACE SCIENCE, ELSEVIER, NL, vol. 133, no. 1, 25 August 2007 (2007-08-25), pages 1-21, XP022214853, ISSN: 0001-8686, DOI: 10.1016/J.CIS.2007.02.003

## Description

The present invention relates to model lipid bilayers and membrane proteins supported therein.

Membrane proteins play a key role in every living cell, which is indicated by the large amount of genes of an organism encoding membrane proteins. 20-30% of the genes of an organism encode for membrane proteins. These proteins are the key factors in the cell's metabolism, for example in cell-cell interaction, signal transduction, and transport of ions and nutrients. Consequently, membrane proteins are the target of about 60% of all pharmaceuticals. However, mechanistic details of membrane proteins are sometimes hard to access, particularly when the role of the membrane/water interface is not negligible. Biomimetic membrane systems have been developed ranging from liposomes into which membrane proteins have been reconstituted to planar lipid bilayers or tethered bilayer lipid membranes.

Contrary to this fundamental role in biology, accessibility of membrane proteins by experimental techniques remains challenging. Structural as well as functional characterization of membrane proteins is difficult due to their amphiphilic properties. An experimental challenge is the sensitivity of membrane proteins to degeneration as soon as they are removed from the native lipid bilayer and solubilized with the help of detergents. To mimic the native lipid environment, solubilized membrane proteins are re-integrated (reconstituted) in artificial lipid bilayers or lipid analogues.

Various model systems of the biological membrane address this issue. Solubilized membrane proteins are purified and reconstituted; i.e., reintegrated in an (artificial) lipid bilayer, which mimics their native environment in the plasma membrane. In the classical liposomal system the lipid bilayer encloses an inner cavity. Therefore, experimental difficulties arise when there is a need to control contents or solute concentrations of the inner compartment. The same holds for the application of a transmembrane potential that is limited to the generation of a diffusion potential by the usage of ion-specific ionophores.

Several reconstitution strategies on solid supports have been applied, such as the insertion into hybrid lipid bilayer membranes of self-assembled monolayers of alkane thiols and phospholipids, tethered lipid bilayer membranes (Knoll et al., Reviews in Molecular Biotechnology 74 (2000) 137), polymer membranes and Langmuir-Blodgett films.

In order to control the orientation of immobilized membrane proteins, Giess (Biophys. J. 87, (2004), 3213-3220) and Ataka et al. (J. Am. Chem. Soc. 2004, 126, 16199-16206) disclose the immobilization of membrane proteins via a His-tag and Ninitrilotriacetic (NTA) moiety on the surface and further the reconstitution of solubilized proteins in the lipid environment by *in situ* dialysis.

Publication WO 2008/118688 A2 discloses a stable supported lipid bilayer membrane, wherein the membrane is stabilized by sterol molecules immobilized to the surface. During manufacture of such a membrane, first sterol molecules are immobilized on the surface and then a membrane is reconstituted with a membrane solution. The membrane solution can contain membrane proteins. These membrane models suffer from low mobility of molecules in the membrane and the lack of any aqueous layer below the membrane, which is usually necessary for native membrane protein activity and behaviour.

JP 2011/027632 A describes a biochip with a lipid bimolecular membrane, that is immobilized onto a substrate with a pattern of hydrophilic and hydrophobic portions on the substrate.

US 2008/0304068 A1 relates to a protein biochip with a layered setup comprising a metallic layer and optionally a lipid double membrane on an active layer. As mentioned for WO 2008/118688 A2 this setup is not suitable to study membrane proteins and suffers from low mobility of proteins and the lack of an aqueous layer.

WO 99/10522 A1 relates to differing artificial membranes based on phosphatidylcholine, phosphatidylserine, phosphatidylethanolamine, and sphingomyelin and tests for assaying binding behaviour of pharmaceutical compounds. Its disclosure lacks membrane protein integration into the membrane.

WO 9610178 A1 relates to a method of inserting membrane proteins from a micelle or vesicle into a planar lipid membrane by fusing the micelle or vesicle with the membrane, wherein the fusion is mediated by functional groups of the membrane that can covalently bind to the micelle or vesicle.

US 7,939,270 relates to probing a planar lipid membrane on a solid surface with a membrane protein, such as a pore or a channel thereby inserting the protein into the membrane, and detecting the insertion. US 6,440,736 provides a further method of artificially inserting membrane proteins into cells or cell membranes.

CA 1243946 A1 describes an antigen/liposome conjugate, wherein a protein antigen is covalently bound to a lipid molecule of the liposome vesicle. WO 01/06007 A2 provides a system of microbubbles for studying and characterizing receptors. Liposome vesicles and microbubbles suffer from lower stability than solid surface bound membranes, especially during manipulation of membrane proteins.

WO 2009/117370 A1 describes membrane coated particles. The membrane is a native cell membrane comprising membrane proteins, at least in part in the natural orientation as found in a cell. The membrane coated particles are described as a batch of similar or nearly identical cell membrane displays suitable for high throughput readouts, in particular in ligand binding assays. However the use of cell membranes, in this case of CHO cells, is time consuming, requires the isolation of biological cell membrane, which can cause unwanted influences of membrane components in an assay of a different protein of interest. Furthermore the membranes are not stabilized on the particles, which can cause further assay imprecisions.

US 7,205,099 B2 relates to a method of studying potassium ion channels in an artificial lipid bilayer, coated onto a flat solid support.

WO 2002/072873 A1 describes the immobilization of proteins onto a sensor surface suitable for surface plasmon resonance spectroscopy, wherein a lipid membrane is reconstituted onto the surface embedding the pre-immobilized membrane protein. The lipid molecules of the membrane are deposited as detergent suspended micelles by slow removal of the detergent molecules. A similar system is described in WO 2010/091293 A1, wherein further Ag particles are deposited onto the surface before membrane coating.

WO 2007/122259 A1 provides surface functionalized gold nanoparticles with covalently attached spacer molecules being themselves linked to proteins in a stereo-specific manner, ensuring controlled orientation of the particle-bound protein.

WO 02/056831 A2, WO 01/49265 A1, and the publications Mirzabekov et al. Nature Biotechnology 200(18): 649 - 654 (2000), Babcock et al. JBC 276(42): 38433-38440 (2001) and Grundner et al. Journal of Virology 76(7):3511-3521 (2002) describe spherical or ellipsoid paramagnetic proteoliposomes containing pure, native, and oriented seven-transmembrane segment immunogenic proteins, such as HIV surface proteins CCR5 or CXCR4. The immunogenic protein is bound onto paramagnetic particles via antibodies.

Nordlund et al., ACS NANO 3 (9) (2009): 2639-2646, relates to porous silica particles enveloped by a lipid bilayer. During manufacture in order to create the lipid envelope, silica particles are treated with vesicles, which open upon contact with the particle and form a layer on the particle.

Sharma et al., Bioconjugate Chemistry 15 (4) (2004): 942-947, describes biotin-PEG3400-bacteriorhodopsin conjugates, which are immobilized on streptavidin coated silica microspheres

Zhong et al., Langmuir 29 (1) (2013): 299-307, describes silica microspheres, modified with a PEG linker (i.e. NHS-PEG₃₀₀₀-NHS) linked to a membrane anchor domain peptide K₃A₄L₂A₇L₂A₃K₂-FITC.

It is a goal of the present invention to provide improved model lipid membranes comprising membrane proteins, that allow quick and reliable testing of the activity of said proteins within the membrane environment.

This goal is achieved by the subject matter of the claims. In particular, the present invention provides a nano- or microsized particle comprising membrane proteins immobilized to the surface of the particle and a sheet of a lipid bilayer interspaced between the membrane proteins and enveloping said particle, wherein said membrane protein is immobilized to the surface of the particle by a linker molecule with a length of at most 5.5 nm in stretched configuration, said particle comprising an aqueous layer between the lipid bilayer envelope and the particle surface, and wherein the membrane proteins within the lipid bilayer are consistently oriented with respect to the particle surface, further comprising an anchor molecule or group attached to the lipid layer attaching the particle to a surface, preferably a surface of a microtiter plate well.. The present invention and its preferred embodiments are further defined in the claims. It is noted that various structural components and parameters can be modified as is explained with regard to preferred embodiments in the following wherein of course each of these embodiments can be combined with other preferred embodiments as should be clear to a skilled person in the art.

According to the present invention, it was found that stable lipid particles can be formed using membrane proteins as anchors for a lipid bilayer.

The selection of relatively short linker molecules, e.g. with a length of at most 5.5 nm (short as compared to an antibody linker as disclosed in WO 02/056831 A2, which has a length of 12 nm) further achieves the benefit of a controlled environment that allows the construction of a balanced lipid membrane with improved membrane fluidity and stability without the need of an additional binding motif of the lipid molecules, which in turn allows improved and reproducible protein activity measurements. As a further advantage, the linker moieties are not only limited in length, they also have smaller lateral dimensions (considerably smaller than bound proteins) so as to allow water molecules to enter the space between the particle and the protein - as well as the lipid layer, which is advantageous for all membrane proteins with a "intracellular portion" (that can be placed in the volume between the particle and the lipid layer) and is a necessary prerequisite for ion transport and hence assessment of activity of ion channels as membrane proteins. Thus also ions can be introduced in a controlled way into the volume between the particle surface and the lipid layer. Furthermore small organic linkers allow the use of well-established protein tag technology, e.g. the Strep-tag or His-tag technology to efficiently immobilize membrane proteins. Combinations of such tags can be further used to control the concentration of two or more membrane proteins in a defined manner.

In addition, the inventive small linker molecules provide increased control of the orientation of the substantially larger membrane proteins. According to the invention, uniform binding and orientation of the membrane proteins is possible, which increases membrane stability. The increased stability also allows reconstitution with water after freezing.

The length of a linker molecule can be estimated by a stretched configuration of the linker molecule as visualized e.g. in a space filling model of the linker molecule (e.g. as shown for proteins in Nowak et al. J. of Solid State Electrochemistry, (2011) 15:105-114, and for lipids in Naumann et al., Langmuir 2003,19,5435-5443). Preferred lengths are at most 5.25 nm, at most 5 nm, at most 4.75 nm, at most 4.5 nm, at most 4.25 nm, at most 4 nm, at most 3.75 nm, at most 3.5 nm, at most 3.25 nm or at most 3 nm. The lateral dimension of the linker molecule is preferably at most 3nm, preferably at most 2.5 nm, especially preferred at most 2 nm, even more preferred at most 1.5 nm, particularly preferred at most 1 nm or even at most 0.75 nm. Smaller lateral dimension allow increased water access into the volume between the particle surface and the lipid layer. Any of these lateral dimensions can be combined with any one of the lengths mentioned above, e.g. linker molecules with a length of at most 5 nm or at most 6 nm and a lateral dimension of at most 1 nm. The length defines the distance to the membrane protein, together with the lateral dimension perpendicular to the length, the volume taken by the linker molecule. The lateral dimension is smaller than the length.

Preferably the particle comprising small spacer moieties permits to accommodate an aqueous layer between particle surface and protein/lipid layer, necessary for a stable bilayer lipid membrane. Preferably the particle comprises a hydrophilic, preferably aqueous, solvent between the lipid bilayer envelope and the particle surface. The inventive enveloped particle can be used to study membrane proteins in a native environment, wherein the volume inside the lipid bilayer simulates a cell or cellular compartment. For such studies, the membrane protein is preferably oriented inside to outside as in a natural environment. Such an orientation can be controlled by consistently immobilizing the membrane proteins on one side (e.g. the cytosolic side). Of course, it is also possible to immobilize the membrane proteins on the other side of the transmembrane domain of the protein, e.g. "inside-out".

The inventive particle is nano- or micrometer sized, which allows stable enveloping by a lipid bilayer. In preferred embodiments the size of the particle is within the range of 5 nm to 1 mm, preferably 30 nm to 900 µm, or 200 nm to 800 µm, preferably 800 nm to 600 µm, especially preferred 1.5 µm to 500 µm, even more preferred 10 µm to 200 µm. An especially preferred range is 5 nm to 100 nm, in particular 10 nm to 60 nm, which lead to optical effects pertinent to nanoparticles such as autofluorescence or spectral changes due to binding of ligands.

Preferably said linker has a length of at least 0.5 nm, preferably at least 1 nm, especially preferred at least 1.2 nm, least 1.4 nm, least 1.6 nm, least 1.8 nm or least 2 nm. Longer linkers allow a safer distance of the membrane protein from the particle surface which may or may not interact with the membrane protein, depending on the partial volume of the protein between the lipid bilayer and the particle (i.e. outside of the membrane, facing the particle). Such an interaction can be prevented by longer linkers. On the other hand, too long linkers may compromise the stability of the entire structure, see above. Therefore, the relative dimension of linker and protein are of paramount significance for the stability of the entire structure as well as the function of the protein incorporated.

Example linkers are organic molecules with a length of e.g. C₁-C₆₀ in a directly connected chain (excluding side chains). "Cₓ" refers to an organic molecule chain of length X (not counting H). Cₓ is an indicator of length and shall not be construed as being limited to carbon atoms. Preferred lengths include C₂-C₅₀, C₃-C₄₀, C₄-C₃₅, C₅-C₃₀, C₆-C₂₈, C₇-C₂₆, C₈-C₂₄, C₉-C₂₂, C₁₀-C₂₀. Such a chain may have one or more heteroatoms, preferably selected from O, N, S, P, Si. In preferred embodiments said organic molecule chain comprises C, especially preferred to a contents of at least 50%, preferably at least 70%. Such a chain may have at least every second atom being C. Further linker molecules include peptides and polynucleotides, especially DNA.

Preferably the membrane protein is immobilized to the particle surface via the linker by a covalent bond, by complex formation, or by adhesion to the linker molecule. Said linker in turn may be immobilized to the surface by a covalent bond, by complex formation, electrostatic attraction or by adhesion, preferably by a covalent bond. A preferred bond is a Ni-NTA (preferably on the linker) to His-tag (preferably on the protein) bond or the strep-tag bond, which are examples of complex formations. Other bond types include azomethine (Schiff base) bonds or diimine bonds.

It is also possible to immobilize additional spacer molecules to the particle surface. Spacer molecules do not bind to a membrane protein and can be used to regulate the surface density of surface-bound linker molecules. Spacer molecules can be selected from the same type of molecules as the linkers, but are not bound to a membrane protein. E.g. during manufacture a preselected linker/spacer ratio can be used to obtain a desired surface density of linker molecules. In turn, this affects the surface density of membrane proteins. In addition or alternatively, membrane protein surface density, i.e. the amount of immobilized membrane proteins per surface area, can be regulated by blocking a preselected percentage of linker molecules during manufacture. Thereby potential linker molecules can be converted into spacer molecules. Such a blocking reaction can be performed by binding inert molecules to the protein reactive groups of the linker, e.g. by binding small peptides or non-membrane proteins.

An example linker is dithiobis (nitriloacetic acid butylamidyl propionate (DTNTA), Nowak et al. J. of Solid State Electrochemistry, (2011) 15:105-114. Preferably the surface comprises further Inert spacer molecules, such as dithiopropionic acid (DTP). These spacer molecules are inert with regard to a membrane protein binding reaction - as compared to the linkers, which bind such membrane proteins during manufacture of the inventive particles. The spaces cover the surface of the particle in the areas between the linkers. The dithio- or any other thio-functionality of the spacer and/or the linker can be used to immobilize the spacer and/or linker on metal surfaces, e.g. an Au-surface. Of course other functionalities can be used to immobilize the spacer and/or linker to other surfaces. It is also possible to convert inert molecules into linker on the particle surface, e.g. by binding a protein binding moiety to an active ester as described in Friedrich et al., Journal of Physical Chemistry B (2008), 112(10), 3193-3201.

In preferred embodiments the linker to spacer ratio is from 1:10 to 5:1, preferably from 1:6 to 2:1, especially from 1:4 to 1.5:1. It has been found that the optimal linker density may vary dependent on the size of the membrane protein. For large membrane proteins it is preferred to have a linker:spacer ratio of 1:4. For smaller membrane proteins, e.g. somatostatin, this ratio can be e.g. 1:1. In related embodiments preferably between 10% to 80%, especially preferred between 12% and 70%, even more preferred between 15% and 60%, in particular preferred between 20 % and 50%, of the surface of the lipid envelope is constituted by a membrane protein. Suitable densities, also known as packing densities can be selected as is known in the art (Friedrich et al., J. Phys. Chem. 2008,112,3193-3201; Schmidt et al., Biosensors & Bioelectronics 13 (1998) 585-591; all incorporated herein by reference). Packing densities can be determined as described by Kunze et al. Langmuir 2006,22,5509-5519, and modified to obtain optimized protein activities (Friedrich et al., J. Phys. Chem. B2008,112,3193-3201; Nowak et al. J. of Solid State Electrochemistry, (2011) 15:105-114).

Preferred linker-protein binding functionalities or protein binding moieties include ACP-Tag, BCCP, c-myc-Tag, Calmodulin-Tag (CaM-Tag), CBP-Tag, Chitin-Tag, FLAG-Tag, HA-Tag, poly-Histidin-Tag (His-Tag), Maltose binding protein-Tag (MBP-Tag), Nus-Tag, S-Tag, Snap-Tag, Streptavidin-Tag (Strep-Tag), Tandem-Affinity-Purification-Tag (TAP-Tag), Thioredoxin-Tag. Similar bonds can be used to bind the linker to the particle surface, especially active ester bonds (e.g. to COOH or NH₂ functional groups), carbodiimides. It is possible to use two or more of such linker-protein binding functionalities to immobilize two or more different membrane proteins in a controlled fashion. Accordingly, it is possible to exactly regulate to concentration of the two or more membrane proteins, which can be different from each other.

Preferably the particle is dispersed in a hydrophilic, preferably aqueous, medium. Usually membrane proteins are studied in aqueous fluids, which may mimic natural environments of cells or cellular compartments.

Preferably the particle comprises a hydrophilic surface. If the particle surface material is not hydrophilic per se, it can be modified by spacer molecules to become hydrophilic, in order to avoid hydrophobic interaction with the surface and to facilitate the formation of an aqueous submembrane space.

According to preferred embodiments the particle is of or comprises a material selected from a polymer, preferably a carbohydrate polymer, especially polysaccharide, a metal or metalloid, preferably Ag, Au, Pd, Pt, Fe, Ni, Si, Ti, Ta, GeAs, or a carbon nanoparticle, peptides or combinations thereof, such as polyaminosaccharide. Special preferred materials include crosslinked or non-crosslinked polymers of a pentose or hexose, such as glucose, galactose, fructose, mannose, or amines thereof. Especially preferred, the polymer material comprises agarose. Preferably the particle is a hydrogel (Kibrom et al. Soft Matter, 2011,7, 237-246). A preferred polyaminosaccharide is chitosan. Peptide materials may comprise collagen, e.g. gelatine. Further examples of polymers include polystyrole, PMMA, POM, PVP. Carbon nanoparticles are e.g. C-nanotubes, HOPG, fullerene. Combinations of these materials include particles with a coreshell structure, e.g. a core of one material with a surrounding shell of a different material. Material combinations include quantum dots.

The particle can have any body shape, including spheres, ellipsoids, sickles, polyhedrons, cylinders, or rods, with any profile such as circles, triangles, polygons.

The inventive particle may comprise one or more further membrane protein, different from said immobilized membrane protein, in said lipid bilayer. Such further membrane proteins may or may not function as scaffold for establishing the lipid bilayer on the particle, but may as well be the object of investigations, e.g. as described in more detail below (e.g. activity or ligand binding assays), similar as the membrane protein bound to the linkers. In the case of more than one membrane protein, one of the membrane proteins (e.g. the immobilized one) may be inert to a reaction under investigation or it may interact with the other membrane protein or with a reaction product of the other membrane protein. An example of a co-reconstitution of two interacting membrane proteins is shown in the examples section below.

The inventive particle may also comprise a further protein adhered to said lipid bilayer. Said adhered protein may not be a membrane protein, but it is a protein anchored in, e.g. by a lipid molecule, or bound to the lipid membrane of the inventive particle. Such proteins can be bound to a membrane protein or a lipid molecule of the lipid membrane or they can be bound to the membrane via lipid anchors. Such adhered proteins may, similar to further membrane proteins, be an object for reaction studies as further detailed below. In the case of more than one protein, one of the proteins (e.g. the immobilized membrane protein) may be inert to a reaction under investigation or it may interact with the other protein or with a reaction product of the other protein.

Preferably the lipid bilayer is a membrane of two layers of amphiphilic molecules, preferably wherein said molecules comprise a hydrophobic portion of a size of C₆ to C₃₀, preferably C₈ to C₂₆, especially preferred C₁₀ to C₂₂ or C₁₂ to C₂₄, or any ranges in between these sizes. Preferred are phospholipids or steroids. Any known lipid bilayer forming molecules can be used, e.g. as described in any one of the references cited herein, which are incorporated herein by reference. Said molecules form a thin membrane enveloping the particle with the membrane protein acting as anchor to tether the membrane to the particle. Usually the membrane protein has a transmembrane domain to which lipids align and form the lipid bilayer. The lipid bilayer is also referred to as lipid membrane herein. The thickness of the lipid bilayer depends on the used lipid molecules and is usually within the range of 3 to 12 nm, preferably 4 to 6 nm.

Other anchor molecules or groups can be attached to the lipid layer that allow attachment of the lipid enveloped particle to any surface, such as a pyrene group particularly designed for hydrophobic surfaces, or a streptavidine/avidin anchor group, which makes the enveloped particles particularly designed for use in microtiter plate wells.

According to the invention, the membrane protein functions as anchor as starting molecule to establish a lipid bilayer at a distance from the particle surface dependent on the linker molecule. The lipid molecule may not have any further attachment to the particle surface. The membrane protein (or group of membrane proteins) may be the only molecule binding the lipid to the particle.

A membrane protein within the lipid bilayer, be it immobilized or not immobilized to the surface of the particle, or the protein adhered to said lipid bilayer can be selected from an integrin, ion channel, transporter protein, a membrane receptor, a peripheral protein, a membrane-associated protein, a redox-protein or combinations thereof, especially membrane protein complexes, a G-protein coupled protein, especially a G-protein receptor. As said, the inventive small linker molecules are particularly advantageous to establish a water volume between the particle surface and the lipid layer, which is especially useful to model ion channel environments and to assay for ion channel activities. The inventive particles can be used for testing, e.g. by high throughput screening, ion channels by fluorescence, especially FRET assays (Jesus et al. DDT Vol. 4, No. 9, 1999: 431)

The membrane protein can be an isolated protein or a protein in protein complex. Membrane protein complexes include without limitation any protein complex in Cytochrome c metabolism, e.g. a complex of coenzyme Q with cytochrome c reductase, or cytochrome c oxidase complex or a photosynthetic reaction center complex, e.g. photosystem I, photosystem II or a cytochrome b complex. Especially preferred are cation channels such as H⁺, K⁺, Na⁺ or Ca²⁺ ion channels (Naumann et al. Journal of Electroanalytical Chemistry 550-551 (2003): 241-252), anion channels, such as a Cl⁻ channel, or channelrhodopsin (M. Nack et al. FEBS Letters 586 (2012) 1344-1348), or g-coupled proteins, e.g. rhodopsin (Kirchberg et al, PNAS Early Edition, doi/10.1073/pnas.1015461108), or acetylcholine receptor (Schmidt et al., Biosensors & Bioelectronics 13 (1998) 585-591). Other membrane proteins include receptors, in particular bacterial or viral surface receptors (Babcock et al. JBC 276(42): 38433-38440 (2001); Grundner et al. Journal of Virology 76(7):3511-3521 (2002)).

The invention further relates to a method of manufacture of a particle with a lipid bilayer envelope according to the invention, comprising the steps of providing a nano- or microsized particle, immobilizing membrane proteins to the surface of the particle, adding amphiphilic molecules suitable to form a lipid bilayer, thus providing particles with said lipid bilayer envelope, and attaching the lipid enveloped particles with an anchor molecule or group, which is attached to the lipid bilayer, to a surface. The membrane proteins are immobilized via linkers-binding as described above. During the step of binding of the membrane protein to the linker, the protein may be stabilized by a detergent (in the absence of the lipid membrane), in a next step, amphiphilic molecules, also referred to as lipids herein, are then deposited to form the lipid bilayer. The lipids may be provided in solution stabilized by detergents. By dialysis of the detergent molecules, the lipid membrane gradually forms as is known in the art (e.g. Friedrich et al., J. Phys. Chem. 2008,112,3193-3201; Naumann et al., Soft Matter, 2011, 7, 9535; Ataka et al., J. AM. CHEM. SOC. 2004, 126, 16199-16206; Giess et al., Biophysical Journal 87, 2004: 3213-3220; all incorporated herein by reference).

The invention further provides a method of testing a biological activity of a membrane protein, comprising providing a particle according to the invention and assaying for the biological activity of a membrane protein within the lipid bilayer enveloping the particle.

Biological activities can be tested as is generally known in the art (e.g. Friedrich et al., Biophysical Journal 95, 2008: 1500-1510). Chemical reactions or binding reactions can be performed, which lead to a detectable signal in dependence of said reaction. Chemical reactions include enzymatic reactions of an enzyme, binding of a ligand to a protein, especially a receptor protein but also any other protein. Accordingly it is possible to screen for potential inhibitor or activator compounds. Accordingly, the invention also relates to a method of assaying a membrane protein for its capability of binding a candidate binding substance, comprising providing a particle according to the invention, adding a candidate binding substance and determining binding events of said candidate binding substance and the membrane proteins in the lipid bilayer enveloping the particle. Further activities include the change of an ion concentration in the hydrophilic, preferably aqueous layer, below the lipid membrane of the particle, in particular in case of ion channels, which may modify the ion concentration dependent on their activity.

The biological activity may be selected from an enzymatic reaction, transportation of a molecule or ion, preferably transportation of an ion by an ion channel, and binding of a ligand. An enzymatic reaction is a reaction catalyzed by the protein converting on or more substrates to one or more products differing from the substrates. Transportation involves the transport of a moiety from one side of the membrane to the other side of the membrane. Usually, the transported moiety passes and interacts with the protein, especially on a surface of the protein. The surface may be an interior surface, as e.g. in a channel. Binding of a ligand may or may not involve a natural ligand, i.e. a ligand that is a binding partner of the protein in a biological system according to the natural function of the protein, e.g. a receptor and a signaling molecule.

Any such activity, reaction, binding event or concentration change can be determined by known means including fluorescence or CD spectroscopy, UV spectroscopy, VIS spectroscopy, NIR or IR transmission, electrostatic, e.g. changes in the membrane potential, pH changes, trans-membrane potential changes. A further very important parameter for quantitative assays of ion channels is the formation of a membrane potential (see further below) associated with ion transport, accessible by fluorescence particularly FRET measurements and particularly designed for high throughput screening (Jesus 1999, supra). E.g. with a metal particle, such as an Ag or Au particle, it is possible to use the optical properties of the system to determine ion concentration, e.g. pH, changes, associations or dissociations of compounds from the membrane system, in particular from the membrane or the membrane protein.

Preferably said candidate binding substance is a candidate active substance potentially modifying a biological activity of said membrane protein, wherein the step of determining binding events comprises the step of determining a biological activity of interest of said membrane protein. Such biological activities have been discussed above and include trans-membrane potential changes of the lipid bilayer, or in the volume between the particle and the lipid bilayer (especially for ion channels) or catalytical activities (especially for enzymes) and binding events (e.g. for receptors, which bind a specific ligand, in particular a natural ligand, which may be competitively inhibited from binding due to the presence of the candidate). Biological activities may be triggered by various conditions, such as in the case of ion channels, where activity can be triggered by a change in ion concentration. Such biological activities may be modified by the candidate compound, e.g. opening/closing of the channel at higher or lower ion concentrations. Other modes of activation may include light excitation, application of a trans-membrane potential or presence of a substrate.

The invention further relates to a method of preparing a durable reconstitutable preparation of particles with a lipid bilayer, comprising providing particles according to the invention and freezing said particles, preferably shock-freezing said particles. The inventive particles have demonstrated a good stability and can be frozen for long-term storage, e.g. for storage of at least 2 months, at least 3 months, at least 4 months, at least 5 months, at least 6 months, at least 8 months, at least 10 months, or at least 12 months. After storage, the particles can be thawed and/or restored to any medium that is of interest, in particular an aqueous medium. Usually the particles are used dispersed in aqueous fluids.

The present invention is further described in the figures and following examples, without being limited to these embodiments of the invention.

### Figures:

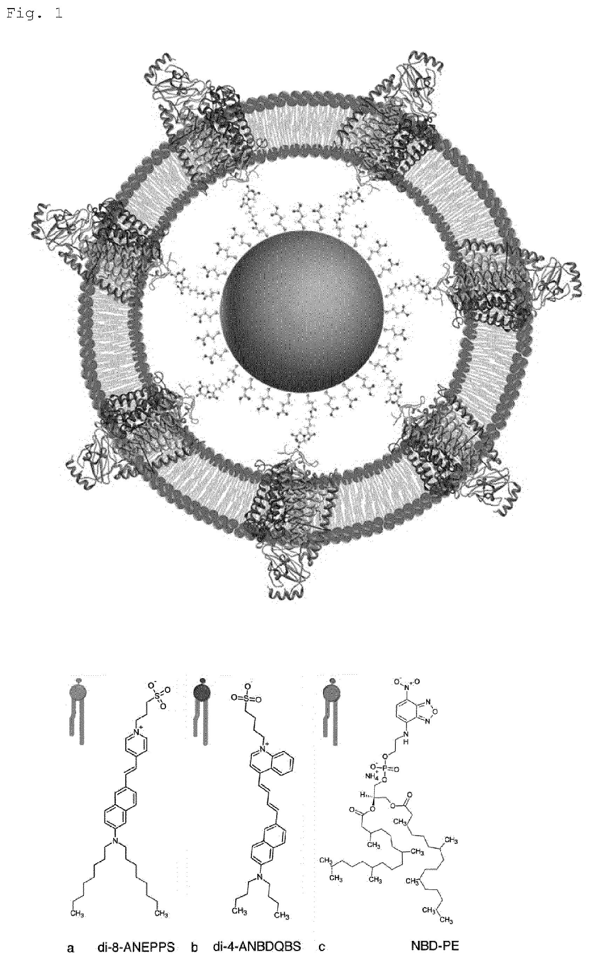

Fig. 1: Schematics of a Proteo-Lipobead (PLB) based an NTA modified agarose bead (central sphere with C₉-NTA linkers) with CcO (indicated as helical structure in the membrane) immobilized in strict orientation via a His-tag attached to subunit I. DphyPC molecules, marked in yellow and red are inserted in between the proteins. Different membrane bound fluorescent labels are inserted into the lipid phase; a) 4-(1-[2-(di-n-octylamino)-6-naphthyl]-2-ethenyl)-1-(3-propylsulfonate) pyridinium betaine (di-8-ANEPPS); b) 4-(1-[2-(di-n-butylamino)-6-naphthyl]-4-butadienyl)-1-(4-butyllsulfonate) quinolinium betaine (di-4-ANBDQBS) c) 1,2-dihexadecanoyl-sn-glycero-3-phospho-(N-[4-nitrobenz-2-oxa-1,3-diazolyl)ethanolamine (NBD-PE). Pyrene lipid anchors can be used to anchor the lipid to the detection substrate such microtiter plates.

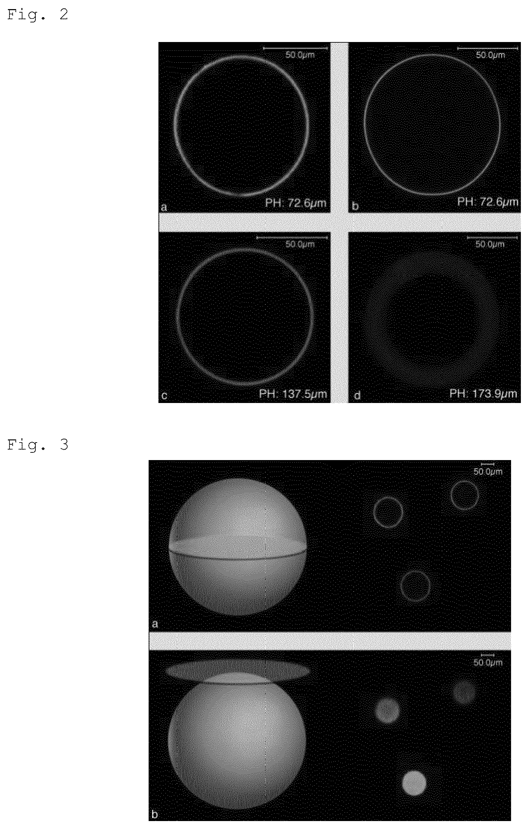

Fig. 2: Laser scanning images of a CcO based PLB labelled with a) NBD PE b) Di-8-ANEPPS and c) di-4-ANBDQBS. d) lipo-beads labelled with di-4-ANBDQBS in the absence of CcO.

Fig. 3: Laser scanning images taken at the a) equatorial plane and b) the pole of a CcO based PLB labelled with di-8-ANEPPS.

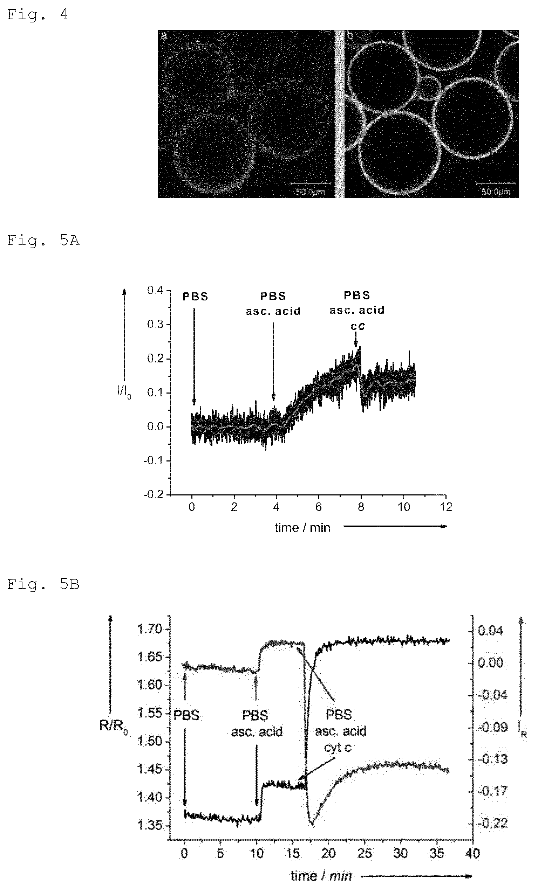

Fig. 4: Laser scanning images taken at the equatorial plane of CcO based PLBs with a primary antibody specifically bound to subunit 1 and subunit II of CcO. A Cy5 conjugated secondary antibody was used as a fluorescent label a) in the absence and b) presence of di-4-ANBDQBS in the lipid phase.

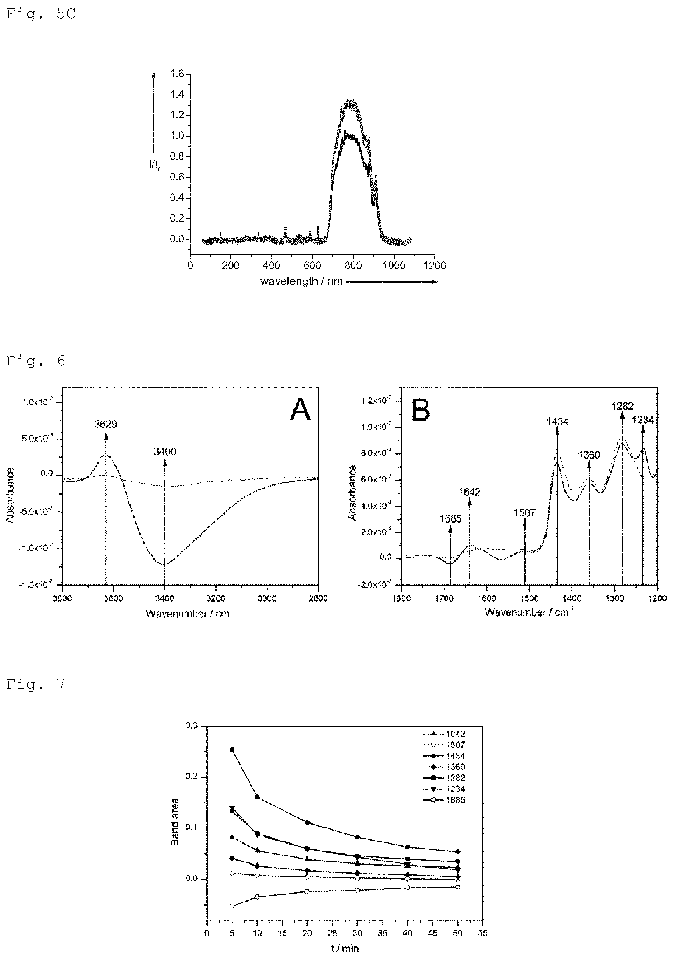

Fig. 5: Time dependent change of the relative fluorescence intensity I/I₀ of membrane bound di-4-ANBDQBS after addition of cytochrome c to the ascorbic acid containing solution by A) laser scanning confocal microscopy (LSM), the middle solid line is to guide the eye, and B) by surface plasmon enhanced fluorescence spectroscopy (SPFS). The SPFS measurement also shows the relative shift of the SPR angle (black line, increasing from R/R₀ of 1.37 to 1.65). C) Fluorescence spectra of di-4- ANBDQBS labeled Proteo-Lipo-Beads after addition of PBS, ascorbic acid and cytochrome c. Spectra are normalized to the maximum of the PBS-spectra.

Fig. 6: Steady-state light-minus-dark spectra of only RC (blue line) and co-reconstituted with *b*c₁ complex (orange line) in the ptBLM in the upper (A) and lower (B) wavenumber region.

Fig. 7: Kinetics of characteristic bands during relaxation. Full circles 1434 cm⁻¹ band, full squares 1282 cm⁻¹ band, full up triangles 1642 cm⁻¹ band, full down triangles 1234 cm⁻¹ band, full diamonds 1360 cm⁻¹ band, empty circles 1507 cm⁻¹ band, empty squares 1685 cm⁻¹ band. All bands relax regularly while the band at 1234 cm⁻¹ has a relaxation pattern different from the other bands.

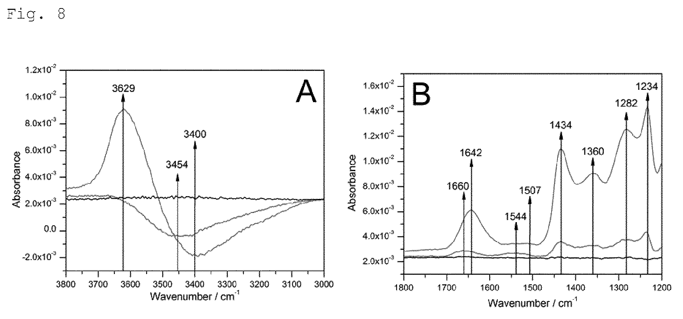

Fig. 8: Steady-state light-minus-dark spectra of RC co-reconstituted with *bc*₁ complex and additional Q₁₀ in the ptBLM before (green line) and after addition of cytochrome c (red line) in the upper (A) and lower (B) wavenumber region. The reference spectrum (black line) was recorded in the dark prior to illumination.

### Examples:

### Example 1: Materials

Ultrapure water (18.2 MΩcm) was used from a water purification system (arium pro UV, Sartorius Stedim Biotech GmbH, Göttingen, Germany). Potassium chloride (KCl, ≥99%), potassium phosphate dibasic (K₂HPO₄, puriss.), dodecyl-β-D-maltoside (DDM, ≥98%), cytochrome c from bovine heart (cyt c, >95%) were purchased from Sigma-Aldrich. L(+)-ascorbic acid ( ≥99%) was purchased from Carl-Roth. 1,2-diphytanoyl-sn-glycero-3-phosphocholine (DiPhyPC, >99%) and 1,2-dioleoyl-sn-glycero-3-phosphoethanolamine-N-(1-pyrenesulfonyl) Pyrene-DOPE (Pyrene, >99%) were purchased from Avanti Polar Lipids. The potential sensitive fluorescence dye di-8-ANEPPS (di-8-butyl-amino-naphtyl-ethyl-ene-pyridinium-propyl-sulfonate), di-4-ANBDQBS (4-(1-[2-(di-*n*-butylamino)-6-naphthyl]-4-butadienyl)-1-(4-butyllsulfonate) quinolinium betaine), and the phospholipid NBD-PE (1,2-dihexadecanoyl-sn-glycero-3-phospho-(N-[4-nitrobenz-2-oxa-1,3-diazolyl)ethanolamine) were purchased from Invitrogen. Cytochrome c oxidase (CcO) from Paracoccus denitrificans with a His-tag engineered to the C-terminus of the subunit I was expressed and purified according to Dürr et al. (Journal of Molecular Biology 2008, 384, 865-877).

The polyclonal primary rabbit antibody, applied here as an enriched IgG fraction reacting specifically with subunit I and II, was obtained after immunization with a native CcO preparation. The secondary antibody, goat anti-Rabbit IgG H&L (Cy5), was purchased from Abcam. Agarose beads, HisPur Ni-NTA Resin, 50-150µm were purchased from Thermo Scientific. Gold granules (99.99%) for evaporation were purchased from Mateck GmbH (Juelich, Germany). di-8-ANBDQBS was synthesized according to Wuskell et al. (Journal of Neuroscience Methods 2006, 151, 200-215). SU8 photoresist was purchased from microchem.

### Example 2: Preparation of the Proteo-Lipo-Beads (PLBs) loaded with Cytochrome c oxidase:

0.5 mL of the HisPur Ni-NTA Resin, slurried in 20% ethyl alcohol were repeatedly rinsed, centrifuged (Heraeus Fresco Microcentrifuge, Thermo Scientific) for 1min at 5x10³ rpm and resuspended, first in ultrapure water, then DPK buffer solution (0.05 M K₂HPO₄, 0.1 M KCl, pH=8) and finally in DDM DPK buffer (0.05 M K₂HPO₄, 0.1 M KCl, pH=8, 0.1% DDM). Thereafter, CcO dissolved in DDM DPK buffer was adsorbed to the Ni-NTA-functionalized surface at a final concentration of 197 nM. After 2h adsorption time the beads were rinsed again, centrifuged and resuspended in DDM phosphate buffer to remove unspecifically adsorbed proteins.

### Dialysis:

The Spectra/Por Float-A-Lyzer (MWCO: 500-1000Da) (volume 5ml), obtained from Carl Roth was filled with 4 ml of a DiPhyPC-Pyrene DOPE solution (1:10) in DDM-DPK buffer and the agarose beads loaded with CcO. In the case of NBD-PE labeled proteo-lipo-beads, 2.6 µL of a 1.9 mM NBD-PE stock solution was added to the DiPhyPC-Pyrene DOPE solution before filling the Float-A-Lyze to a final NBD-PE concentration of 12.2 µM. The sample was dialyzed in 1 L of DPK buffer solution at room temperature for 24 hours with 6 complete dialysate changes after 1, 2, 4, 14, 18 and 22 hours.

### Labeling with potential-sensitive fluorescent dyes (PSFDs):

Stock solution of PSFD di-4-ANBDQBS was prepared in pure ethanol solution at a concentration of 2 mg/10 mL. The stock solution of di-8-ANEPPS was prepared by dissolving the powder in DMSO at a ratio of 2 mg/10 mL. 20 µL of the stock solution was added to the suspension of PLBs loaded with CcO to a final concentration was 6.6 µM and 6.9 µM di-8-ANEPPS or di-4-ANBDQBS, respectively. After incubation for 15-20 min the PLBs were washed three times in dye-free PBS buffer solution by rinsing, centrifugation and resuspension.

### Example 3: Laser scanning confocal fluorescence microscopy (LSM)

LSM measurements were carried out in an upright Leica TCS SP5 II microscope with a 10x dry objective (Leica, HC PL APO 10x/0.40 CS). The Proteo-lipo-beads were attached via the hydrophobic pyrene anchor group to the hydrophobic surface of the flow cell (µ-slide upright, ibidi GmbH, Munich, Germany), thus withstanding the hydrodynamic stress caused by the flux of aqueous buffer solutions up to a flow rate of of 500 µl/min (IPC, Ismatec, Idex Health & Science group, Glattburg, Switzerland). Either the 458 nm or the 488 nm line of a multi argon laser or alternatively the Red 633 nm line of the He-Ne laser were used for the excitation of, NBD-PE, di-8-ANEPPS or di-4-ANBDQBS, respectively.
In case of di-8-ANEPPS labeled proteo-lipo-beads, measurements were done in the two channel ratiometric mode (550-580 nm and 620-650 nm). Because of the broad emission band of di-4-ANBDQBS, measurements were carried out in the one channel mode (680-720 nm) (Fig. 5C). For background noise analysis in case of di-4-ANBDQBS measurements were also done in the ratiometric mode (630-636 nm and 680-720 nm).

### Example 4: Surface Plasmon enhanced Fluorescence Spectroscopy (SPFS)

SPFS was performed in a custom made setup using the Kretschmann-configuration (Reather, 1977, in: Haas, G., Francombe, M., H., Hoffmann, R., W., (Eds.) Physics of Thin Films, vol. 9. Academic Press, New York, p.145) described. The glass slide (LaSFN9 glass from Hellma Optic, Jena, refractive index n=1.8385 at 633 nm) coated with 43 nm gold (HHV Edwards Auto 306, Crawley, UK) was optically matched to the base of a 45° glass prism (LaSFN9). di-4-ANBDQBS labeled Proteo-Lipo-Beads were adhered to the hydrophobic surface of a photoresist film (SU8 from microchem 25 nm) spincoated on top of the gold layer (Spincoat G3, Specialty Coating Systems Inc., Indiana, USA). Monochromatic light from a He/Ne Laser, (Uniphase, San Jose, CA, λ = 632.8 nm) was directed through the prism and detected by a custom made photodiode detector. The fluorescence light emitted from the surface was collected through the flow-cell by a lens (numerical aperture NA 0.3), directed through a notch filter (632.8 nm) and a band-pass filter (transmission wavelength of λ 694/10 nm) and finally detected by a photomultiplier tube (Hamamatsu H6240-01, Japan).In case of SPFS kinetics, reflectivity and fluorescence intensity changes are measured at an angle of incidence θ=65.5° while rinsing 9 min with PBS, 6 min with 3.3 mM PBS-ascorbic acid solution and 20 min with 105 µM PBS-ascorbic acid-cytochrome c solution (Fig. 5B).

### Example 5: Fluorescence spectroscopy

Fluorescence measurements were carried out using a He-Ne Red 632.8 nm, 10 mW laser (1135P, JDS Uniphase Corporation, Milpitas, USA) for excitation of di-4-ANBDQBS labeled Proteo-Lipobeads which are attached to the hydrophobic surface of the flow cell as described above. The emitted fluorescence signal from the surface is collected by the objective and separated from the reflected laser light via a dichroic beam splitter (LC-660DRLP-25, Laser components, Olching, Germany) and a 632.8 nm notchfilter (Rugate Technologies Inc., Oxford, USA). A 10x objective (MPlan N 10x0.25NA, Olympus Corporation, Tokyo, Japan) is also used. Fluorescence spectra are measured by a spectrograph (Andor Shamrock 303i, Andor Technology plc., Belfast, UK) and a CCD spectroscopy detector system (LOT Oriel Newton 920P, LOT Oriel GmbH, Darmstadt, Germany) which is cooled down to -70°C. Due to the low quantum yield of di-8-ANBDQBS data were averaged over 10 spectra each taken at an exposure time of 10 seconds.
Fluorescence spectra were measured after rinsing with each of the following aqueous solutions for 5 min at a flow rate of 500 µl/min: first O₂ saturated PBS buffer solution, second 33 mM ascorbic acid solution in PBS and third 33 mM ascorbic acid with 105 µM cytochrome c solution in PBS (Fig. 5C).

### Time-resolved fluorescence spectroscopy (tr-FS)

In the case of time-resolved fluorescence spectroscopy a 769/41 nm band-pass (Edmund Optics GmbH, Karlsruhe, Germany) was used as an emission filter. The fluorescence signal was detected by a single photon counting module (SPAD) (COUNT 20c-FC, Laser components, Olching, Germany) attached to a 225 MHz universal frequency counter with a gating time of 100 ms (53131A, Agilent Technologies, Boblingen, Germany). Changes in the fluorescence emission photocounts were measured while rinsing 3.5 min with O₂ saturated PBS buffer solution, 4 min with 33 mM ascorbic acid solution in PBS and 3 min with 33 µM ascorbic acid 105 µM cytochrome c solution in PBS (Fig. 5A).

### Example 6 (comparative example): Co-reconstitution of Rhodobacter sphaeroides reaction centers (RC) and bc1 complex on a planar two-layer gold surface designed for surface-enhanced FTIR spectroscopy:

Wild-type RCs with a genetically engineered His-tag at the C-terminus of the M-subunit were expressed and purified from the purple non-sulfur bacterium *Rhodobacter sphaeroides,* the bc1 complex poly-his-tagged on the C-terminal end of the cyt b subunit was expressed and purified according to Crofts et al. (Crofts Protein Expression and Purification 1999, 15, 370). The immobilization of the proteins on the gold surface was performed according to Nowak et al. (Journal of Solid State Electrochemistry 2011, 15, 105). Briefly, the gold surface was immersed in a solution of 10 mM DTNTA and 10 mM DTP at a molar ratio of 0.25 in dry DMSO for 20 h. After rinsing with ethanol and purified water, the surface was immersed in 40 mM NiCl₂ in acetate buffer (50 mM, pH=5.5) for 30 minutes, followed by thorough rinsing with purified water to remove excess NiCl2. The surface was dried under a stream of argon prior to assembly in the measuring cell and rehydrated with DDM phosphate buffer (DDM-DPK) (0.05 M K2HPO₄, 0.1 M KC1, pH=8, 0.1% DDM). RCs and bc1 complexes dissolved in DDM-DPK were adsorbed to the NTA-functionalized gold bead at a final concentration of 100 nM, respectively. After 4 h adsorption time performed at 28 °C, the cell was rinsed with DDM-DPK to remove unspecifically adsorbed and bulk protein. Thereafter DDM-DPK was replaced by a DiPhyPC/DDM-DPK solution (40 µM DiPhyPC in DDM-DPK). In the case of additional ubiquinone, Q10 was solubilized together with DiPhyPC (6 µM Q10 in DiPhyPC/DDM-DPK). DDM was removed by in-situ dialysis by adding biobeads to the DiPhyPC/DDM-DPK solution.

### Example 7 (comparative example): Preparation of the two-layer gold surface on an ATR crystal.

Preparation was done as previously described by Nowak et al. (Applied Spectroscopy 2009, 63, 1068). A polished silicon attenuated total reflection (ATR) crystal was immersed in a 10% ethanolic solution of MPTES for 60 minutes to anchor the gold layer. After rinsing with ethanol, the sample was dried under a stream of argon and annealed at 100 °C for 60 minutes. After cooling to room temperature, the crystal was immersed in water for 10 minutes and dried under a stream of argon. A 25 nm gold film was then deposited onto the ATR crystal by electrochemical evaporation (HHV Edwards Auto 306, Crawley, UK). Gold nanoparticles were grown on the gold film by immersing the crystal in 50 ml of an aqueous solution of hydroxylamine hydrochloride (0.4 mM), to which 500 µl of an aqueous solution of gold(III) chloride hydrate (0.3 mM) was added five times at 2-minutes intervals. Finally, the sample was rinsed with water and dried under a stream of argon.

### Example 8 (comparative example): ATR-SEIRA-Spectroscopy of RC and bc1 complex on Au surface

The electrochemical cell with immobilized RC and *bc*1 complex on Au was mounted on top of a trapezoid single reflection silicon ATR crystal. The IR beam of the FTIR spectrometer (VERTEX 70v, from Bruker, Ettlingen, Germany) was coupled into the crystal at an angle of incidence Θ = 60° by using the custom-made setup described previously (Nowak et al. Appl Spectrosc 2009, 63, 1068). All spectra were measured with parallel polarized light. Because the ATR element surface is coated with an electrical conductor, perpendicularly polarized light is unable to penetrate the conducting layer effectively. The total reflected IR beam intensity was measured with a liquid nitrogen-cooled photovoltaic mercury cadmium telluride (MCT) detector. IR measurements were done under anaerobic conditions at 28 °C. The sample unit was purged with dry, carbon dioxide-free air. FTIR spectra were recorded at 4 cm⁻¹ resolution using Blackham-Harris 3-term apodization and a zero filling factor of 2. The interferograms were measured in double-sided mode and transformed into spectra using the Power phase correction mode. Spectra were analyzed using the software package OPUS 7 and OriginLab's Origin software.

Illumination was performed with white light from a Fiber-Lite DC950 illuminator (150 W, quartz halogen lamp) obtained from Dolan-Jenner (Boxborough, MA) conducted through an optical fiber.

### Example 9: Results - cytochrome c oxidase

Membrane proteins are reconstituted into bilayer lipid membranes to form so-called proteo-lipobeads, PLBs, depicted schematically in figure 1. As a first example nitrilo-tri-acetic acid (NTA) modified micrometer size agarose beads are presented onto which cytochrome c oxidase (CcO) from *P. denitrificans* with the his-tag attached to subunit I is immobilized via his-tag technology. PLBs thus prepared can be conveniently investigated by laser scanning confocal microscopy (LSM) (Claxton, N., S.; Fellers, T. J.; Davidson, M. W. Laser Scanning Confocal Microscopy. Oxford, Bios Scientific Publishers, 1987; Vol. 1979), particularly when the beads are attached to the surface of a substrate. Changes of physical parameters within or in close proximity to the protein/membrane system can be monitored as a function of time using a selection of fluorescent probes. Time-dependent changes can also be detected by surface-plasmon enhanced fluorescence spectroscopy (SPFS).

Oriented immobilization of membrane proteins via his-tag technology is a well-established method. The reconstitution into a bilayer lipid membrane, thereby using the immobilized proteins as a scaffold, had been investigated previously on flat surfaces (Giess et al. Biophys J 2004, 87, 3213; Naumann et al. Soft Matter 2011, 7, 9535; Kibrom et al. Soft Matter, 2011, 7, 237-246). In the case of the micrometer scale gel beads, fluorescence labeled lipids such as NBD PE can be employed to visualize the self-assembled bilayer lipid membranes by LSM (Fig. 2a). NBD PE has been excited by the 458 nm line of the multi-Ar laser. Images were obtained with PLBs into which Di-phytanoyl phospholipid (DPhyPC), NBD-PE and pyrene PC have been co-reconstituted. The hydrophobic pyrene anchor group is designed to attach the beads to the surface of the flow cell. Under these conditions the PLBs adhere to the surface so as to withstand the hydrodynamic stress caused by the flux of an aqueous buffer solution. Moreover, voltage sensitive probes, such as di-8-ANEPPS can be added to the bathing solution. With di-8-ANEPPS excited by the 488 nm line of the Argon laser, the fluorescence spectrum indicates the presence of the bilayer lipid membrane, since only di-8-ANEPPS incorporated into the bilayer lipid membrane gives rise to the detected fluorescence intensity (Fig. 2b). Di-8-ANEPPS unspecifically bound or present within the aqueous bulk phase has a 10⁴ times lower fluorescence intensity. Images shown in Fig. 2a and b illustrate a layer of regular thickness around the bead. However, bilayer lipid membranes are known to self-assemble on silica beads by themselves. Therefore, as a control experiment, gel beads were subjected to dialysis in the absence of immobilized CcO. A bilayer lipid membrane can be seen in the presence of di-8-ANEPPS, however, broader and more blurry than the bilayer lipid membrane in the presence of immobilized membrane proteins (Fig. 2d): Another negative control consisted of trying to label immobilized CcO alone with di-8-ANEPPS. Finally the near-infrared voltage-sensitive fluorescent dye di-4-ANBDQBS was inserted, excited by the HeNe laser, which again indicated a well-defined bilayer lipid membrane surrounding the beads. (Fig. 2c). Images so far, were recorded in the equatorial plane of the beads (Fig. 3a). Laser scanning images with the focal plane adjusted to the top of the PLB showed a continuous fluorescence of smaller diameter as expected for a closed bilayer lipid membrane covering the beads allover (Fig. 3b). From these results it was concluded that using membrane proteins as a scaffold leads to well-defined lipid bilayers evenly covering the entire surface of the bead.

CcO immobilized with the his-tag attached to subunit I is oriented with the cytochrome c binding site pointing to the outside of the membrane. A polyclonal primary IgG-antibody from rabbit specific to both subunit I and II was bound to the PLBs. Subsequently, a Cy5 conjugated secondary antibody was bound to the primary IgG and the Cy5 label was excited by the HeNe laser at 633 nm. The presence of CcO was indicated by a low fluorescence intensity as compared to the intensity obtained using the membrane bound labels (Fig. 4). This indicates a relatively low packing density of proteins embedded into the lipid layer as compared to the lipids.

C*c*O is the terminal complex of the respiratory chain. It converts the free enthalpy gained by the reduction of oxygen to water into a difference in electrochemical potentials of protons, ΔµH̃⁺ across the lipid membrane. The larger part of ΔµH̃⁺ is the membrane potential, ΔΦ. Hence the functionality of CcO within the PLBs may be demonstrated by voltage sensitive dyes. As it is well known from electrometric measurements, ΔΦ is generated in the ms time scale, when CcO is reduced by cytochrome c and ascorbic acid in the presence of oxygen (Belevich et al. Proc. Natl. Acad. Science 2007, 104, 2685). Consequently, ΔΦ was measured under the same conditions, using LSM. Ratiometric measurements using di-8-ANEPPS would be the method of choice, however, the emission spectrum of di-8-ANEPPS shows a significant overlap with the extinction spectrum of cytochrome c in the Q-band region. Moreover, strong electrostatic binding of cytochrome c to CcO may lead to a reduced spatial separation of cytochrome c and di-8-ANEPPS, which could give rise to quenching of the emission of di-8-ANEPPS due to Forster transfer. Electrostatic binding may also affect the dipole potential inside the protein as indicated by the increase of the fluorescence intensity of di-8-ANEPPS which is almost identical no matter whether reduced or oxidized cytochrome c had been bound to the PLBs.

In order to overcome these problems, the lipid membrane was labelled with the near-infrared voltage-sensitive fluorescent dye di-4-ANBDQBS (Loew Bioelectromagnetics, 1992, 13, 179-189; Matiukas et al. American Journal of Physiology-heart and Circulatory Physiology, 2006, 290, H2633-H2643). Di-4-ANBDQBS has a broad emission band with a maximum at ~766 nm, hence the spectral overlap with the extinction of cytochrome c is negligible. The fluorescence emission intensity within the bandwith of 680-720 nm was monitored as a function of time in the equatorial plane of the PLBs. Cytochrome c and ascorbic acid were added to an air saturated solution. Intensity changes were observed in a time scale of minutes, limited by the diffusion of reagents, when only ascorbic acid was added, followed by a fast phase in the time scale of 100 ms, after addition of cytochrome c and ascorbic acid (Fig. 5A). The fast phase was attributed to the generation of a membrane potential as a consequence of proton transfer when CcO is reduced by cytochrome c and ascorbic acid in the presence of oxygen (Belevich et al. Proc. Natl. Acad. Science 2007, 104, 2685.). After the fast phase we observed a second slow phase, which we explained in terms of the slow relaxation of the membrane potential due to passive ion transport - see Fig. 5A (Robertson et al. Journal of Physical Chemistry B (2008), 112(34), 10475-10482).

This result was confirmed by SPFS (Neumann, T.; Johansson, M. L.; Kambhampati, D. & Knoll, W. Advanced Functional Materials, Wiley-v C H Verlag Gmbh, 2002, 12, 575-586). PBLs from CcO with the his-tag attached to subunit I labelled with di-4-ANBDQBS were attached to the hydrophobic surface of a thin photoresist film on top of a 50 nm gold layer. The time dependent change of the SPR resonance angle and the SPF intensity were recorded after addition of ascorbic acid alone and in the presence of cytochrome c. (Fig. 5B). In both cases, a slow phase was observed, when only ascorbic acid was added, followed by a fast phase after addition of ascorbic acid and cytochrome c. The fast phase in the SPFS trace reflects the intensity change due to the generation of the membrane potential, whereas the fast phase in the SPR trace reflects the thickness increase due to cytochrome c binding. This interpretation is consistent with the second slow phase observed but only in the SPFS trace which we had explained above in terms of the slow relaxation of the membrane potential due to passive ion transport.

These results show that CcO has been incorporated in the PBLs in a functionally active form, embedded in a well-defined bilayer lipid membrane, the lipidic components of which are easily interchangeable. Both sides of the protein are accessible, particularly the outer side, as shown by the specific binding of the primary antibody. Functionality of the protein has been demonstrated employing time-resolved LSM as well as SPFS by monitoring the intensity changes of voltage-sensitive fluorescent dyes di-8-ANEPPS and di-4-ANBDQBS inserted in the bilayer lipid membrane after initiating the enzyme cycle with cytochrome c in the presence of ascorbic acid and oxygen. Thus membrane can be incorporated in a functionally active form into the beads and used for ligand binding assays as well as for functionality assays to determine activities of membrane proteins.

### Example 10 (comparative example): Results - Co-reconstitution of photosynthetic reaction centers (RCs) with bc1 complexes on a planar Au layer

Co-immobilization of RCs and *b*c₁ complexes and subsequent formation of the protein-tethered bilayer lipid membrane (ptBLM) followed by SPR and EIS. Optical thickness and electrical parameters correspond to respective data found in the case of CcO. It was concluded that a monolayer of RCs mixed with *b*c₁ complexes had been formed on the gold film, whereas the voids between single proteins are filled with a lipid bilayer.

### Example 11 (comparative example): Light-minus-dark FTIR spectra

Light-minus-dark spectra were recorded and absorbance spectra were calculated using the respective spectra in the dark as a reference. Spectra of the *b*c₁ complex co-reconstituted with the RC are shown in Fig. 6 in comparison with the respective spectra of the RC alone.

In the presence of the *b*c₁ complex, absorbances of the bands at 1282, 1360, 1434 cm⁻¹ are almost unchanged whereas the bands at 1234, 1507, 1642 and the negative band at 1685 cm⁻¹ are considerably decreased. Also the broad negative and positive bands at 3400 and 3629 cm⁻¹ exhibit a smaller absorbance compared to the RC alone. The negative band at 1685 cm⁻¹ accounts for the decrease of the special pair P, whereas the band at 1642 cm⁻¹ is a mixture of the amide I band with H-O-H stretching vibrations of water. The band at 1234 cm⁻¹ is a prominent band, in the region of C-O stretching vibrations of carboxylic acids (Stuart, B. Biological Applications of Infrared Spectroscopy; John Wiley & Sons, Ltd, 1997) and hence could be attributed to the protonation of any COOH group within the protein. The bands at 1282, 1360, 1434 cm⁻¹ have been assigned to the P⁺ species of the special pair, a semiquinone during the transition Q_{A}⁻Q_{B}/Q_{A}Q_{B}⁻, and QH₂, respectively. The broad positive and negative bands at 3400 and 3629 cm⁻¹ had been attributed to water stretching vibrations associated with Q_{A}⁻/Q_{A} as well as Q_{B}⁻/Q_{B} transitions in the RCs.

Thereafter, we have co-reconstituted additional lipophilic Q₁₀ together with the two proteins. All the bands are increased thus confirming the assignments to quinone species. The steady-state obtained after the first illumination was then permitted to relax in the dark. All the bands decrease as shown in some examples in Fig. 7, illustrated in the plots of the absorbances vs. time of relaxation. The remaining bands show a similar behavior, although in a different range of absorbances.

Finally light-minus-dark absorbance spectra were recorded not only in the presence of additional Q₁₀ but also (oxidized) cytochrome c added to the aqueous phase (Fig. 8). This leads to an appreciable decrease of all the bands described above. This decrease indicates a substantial interaction of the light-activated RC with the *b*c₁ complex but only when the electrons delivered to the *b*c₁ complex via QH₂ are used to reduce cc. This is consistent with the decrease of the marker band of QH₂ at 1434 cm⁻¹ providing the reducing equivalents to the *b*c₁ complex, whereas the decrease of the band at 1360 cm⁻¹ assigned to semiquinone species within the RC is consistent with QH₂ formation via semiquinone.
Band assignments are collected in table 1.

**Table 1: Tentative band assignment of the marker bands of RCs co-reconstituted with bc₁ complexes in the ptBLM under continuous illumination.**

| **Band position[cm⁻¹]** | | **Tentative Assignment** | |
|---|---|---|---|
| **Experimental** | **Literature** | **Moiety** | **Vibrational Mode** |
| **1234** | 1232 | Carboxylic acids | C-O stretch |
| **1282** | 1282 | P⁺ | |
| **1360** | 1365, 1355 | | Q_{A}⁻Q_{B}/Q_{A}Q_{B}⁻ |
| **1434** | 1433 | Q_{B}H₂ | |
| **1435** | 1438, 1439 | Semiquinone | |
| **1544** | 1550 | Amide II | |
| **1642** | 1640, 1641, 1642 | Quinone Q_{B} | 1-,4-C=O stretch |
| **1685** | 1682, 1683 | 9-keto group of P | C=O |
| **3400** | 3485 | H₂O | Q_{B}⁻/Q_{B} |
| **3629** | 3632 | H₂O | Q_{B}⁻/Q_{B} |

This shows that membrane protein complex interactions can be modeled in artificial lipid bilayers tethered onto a solid surface, here an Au surface, their reactions observed and analysed, here by ATR-SEIRA-Spectroscopy. Similar results can be expected for any particles with artificial tethered bilayer for modeling the RC and the *b*c₁ complex simultaneously.

## Claims

1. A nano- or microsized particle comprising membrane proteins immobilized to the surface of the particle and a sheet of a lipid bilayer interspaced between the membrane proteins and enveloping said particle, wherein said membrane protein is immobilized to the surface of the particle by a linker molecule with a length of at most 5.5 nm in stretched configuration, said particle comprising an aqueous layer between the lipid bilayer envelope and the particle surface, and wherein the membrane proteins within the lipid bilayer are consistently oriented with respect to the particle surface, further comprising an anchor molecule or group attached to the lipid layer attaching the particle to a surface, preferably a surface of a microtiter plate well.

2. Particle according to claim 1, wherein between 10% to 80% of the surface of the lipid envelope is constituted by a membrane protein.

3. Particle according to claim 1 or 2, wherein the size of the particle is within the range of 5 nm to 1 mm, preferably 30 nm to 900 µm, or 200 nm to 800 µm, preferably 800 nm to 600 µm, especially preferred 1.5 µm to 500 µm, even more preferred 10 µm to 200 µm.

4. Particle according to any one of claims 1 to 3, wherein said linker has a length of at least 0.5 nm, preferably at least 1 nm and/or has a lateral dimension of at most 1 nm.

5. Particle according to any one of claims 1 to 4 dispersed in an aqueous solvent.

6. Particle according to any one of claims 1 to 5, wherein said particle is of or comprises a material selected from a polymer, preferably a carbohydrate polymer, a metal or metalloid, preferably Ag, Au, Si, Ti, Ta, GeAs, or a carbon nanoparticle.

7. Particle according to any one of claims 1 to 6, wherein a further membrane protein, different from said immobilized membrane protein, is comprised in said lipid bilayer.

8. Particle according to any one of claims 1 to 7, wherein said lipid bilayer is a membrane of two layers of amphiphilic molecules, preferably wherein said molecules comprise a hydrophobic portion of a size of C6 to C30, preferably C8 to C26.

9. Particle according to any one of claims 1 to 8, comprising a further protein adhered to said lipid bilayer.

10. Particle according to any one of claims 1 to 9, wherein the membrane protein within the lipid bilayer, immobilized or not immobilized to the surface of the particle, or the protein adhered to said lipid bilayer is selected from an integrin, ion channel, transporter protein, a membrane receptor, a peripheral protein, a membrane-associated protein, a redox-protein.

11. Method of manufacture of a particle with a lipid bilayer envelope according to any one of claims 1 to 10, comprising the steps of providing a nano- or microsized particle, immobilizing membrane proteins to the surface of the particle, adding amphiphilic molecules suitable to form a lipid bilayer, thus providing particles with said lipid bilayer envelope, and attaching the lipid enveloped particles with an anchor molecule or group, which is attached to the lipid bilayer, to a surface.

12. Method of attaching a lipid enveloped particle to a surface comprising
providing a nano- or microsized particle comprising membrane proteins immobilized to the surface of the particle and a sheet of a lipid bilayer interspaced between the membrane proteins and enveloping said particle, wherein said membrane protein is immobilized to the surface of the particle by a linker molecule with a length of at most 5.5 nm in stretched configuration, said particle comprising an aqueous layer between the lipid bilayer envelope and the particle surface, and wherein the membrane proteins within the lipid bilayer are consistently oriented with respect to the particle surface, further comprising an anchor molecule or group attached to the lipid layer suitable for attachment of the particle to a surface, preferably a surface of a microtiter plate well, preferably said particle being further defined as in any one of claims 2 to 10,
and attaching the lipid enveloped particles to the surface.

13. Method of testing a biological activity of a membrane protein, comprising providing a particle according to any one of claims 1 to 10 and assaying for the biological activity of a membrane protein within the lipid bilayer enveloping the particle.

14. Method of assaying a membrane protein for its capability of binding a candidate binding substance, comprising providing a particle according to any one of claims 1 to 10, adding a candidate binding substance and determining binding events of said candidate binding substance and the membrane proteins in the lipid bilayer enveloping the particle.

15. The method of claim 14, wherein said candidate binding substance is a candidate active substance potentially modifying a biological activity of said membrane protein, wherein the step of determining binding events comprises the step of determining a biological activity of interest of said membrane protein.

16. The method of claim 13 or 15, wherein said biological activity is selected from an enzymatic reaction, transportation of a molecule or ion, preferably transportation of an ion by an ion channel, and binding of a ligand.

17. The method of preparing a durable reconstitutable preparation of particles with a lipid bilayer, comprising providing particles according to any one of claims 1 to 10 and freezing said particles, preferably shock-freezing said particles.

## Patentansprüche

1. Partikel in Nano- oder Mikrogröße, umfassend Membranproteine, die an der Oberfläche des Partikels immobilisiert sind, und einen Überzug aus einer Lipiddoppelschicht, der in die Räume zwischen den Membranproteinen eingedrungen ist und das Partikel umhüllt, wobei das Membranprotein an der Oberfläche des Partikels durch ein Linker-Molekül mit einer Länge von höchstens 5,5 nm in ausgestreckter Konfiguration immobilisiert ist, wobei das Partikel eine wässrige Schicht zwischen der Hülle aus der Lipiddoppelschicht und der Partikeloberfläche umfasst und wobei die Membranproteine innerhalb der Lipiddoppelschicht einheitlich orientiert sind mit Bezug zu der Partikeloberfläche, weiterhin umfassend ein an der Lipidschicht angeheftetes Ankermolekül oder Gruppe, das/die das Partikel an einer Oberfläche, vorzugsweise der Oberfläche einer Vertiefung einer Mikrotiterplatte, anlagert.

2. Partikel gemäß Anspruch 1, wobei zwischen 10% und 80% der Oberfläche der Lipidhülle durch ein Membranprotein gegeben ist.

3. Partikel gemäß Anspruch 1 oder 2, wobei die Größe des Partikels innerhalb des Bereichs von 5 nm bis 1 mm, vorzugsweise 30 nm bis 900 µm, oder 200 nm bis 800 µm, vorzugsweise 800 nm bis 600 µm, besonders bevorzugt 1,5 µm bis 500 µm, sogar mehr bevorzugt 10 µm bis 200 µm, liegt.

4. Partikel gemäß einem der Ansprüche 1 bis 3, wobei der Linker eine Länge von mindestens 0,5 nm, vorzugsweise mindestens 1 nm aufweist und/oder eine laterale Dimension von höchstens 1 nm aufweist.

5. Partikel gemäß einem der Ansprüche 1 bis 4, dispergiert in einem wässrigen Lösungsmittel.

6. Partikel gemäß einem der Ansprüche 1 bis 5, wobei das Partikel aus einem Material besteht oder es umfasst, ausgewählt aus einem Polymer, vorzugsweise einem Kohlenhydratpolymer, einem Metall oder Metalloxid, vorzugsweise Ag, Au, Si, Ti, Ta, GeAs, oder einem Kohlenstoff-Nanopartikel.

7. Partikel gemäß einem der Ansprüche 1 bis 6, wobei ein weiteres Membranprotein, das unterschiedlich zu dem immobilisierten Membranprotein ist, in der Lipiddoppelschicht eingeschlossen ist.

8. Partikel gemäß einem der Ansprüche 1 bis 7, wobei die Lipiddoppelschicht eine Membran aus zwei Schichten von amphiphilen Molekülen ist, vorzugsweise wobei diese Moleküle einen hydrophoben Teil einer Größe von C6 bis C30, vorzugsweise C8 bis C26, umfassen.

9. Partikel gemäß einem der Ansprüche 1 bis 8, umfassend ein weiteres, an der Lipiddoppelschicht anhaftendes Protein.

10. Partikel gemäß einem der Ansprüche 1 bis 9, wobei das Membranprotein innerhalb der Lipiddoppelschicht, ob nun an der Oberfläche des Partikels immobilisiert oder nicht immobilisiert, oder das an der Lipiddoppelschicht anhaftende Protein, ausgewählt ist aus einem Integrin, lonenkanalprotein, Transportprotein, einem Membranrezeptor, einem peripheren Protein, einem Membran-assoziierten Protein, einem Redox-Protein.

11. Verfahren zur Herstellung eines Partikels mit einer Hülle aus einer Lipiddoppelschicht gemäß einem der Ansprüche 1 bis 10, umfassend die folgenden Schritte: Bereitstellen eines Partikels in Nano- oder Mikrogröße, Immobilisieren von Membranproteinen an der Oberfläche des Partikels, Hinzufügen von amphiphilen Molekülen, die sich zur Bildung einer Lipiddoppelschicht eignen, damit Versehen der Partikel mit der Hülle aus einer Lipiddoppelschicht und Anlagern der Lipid-umhüllten Partikel mit einem Ankermolekül oder einer Gruppe, das/die an die Lipiddoppelschicht angeheftet ist, an einer Oberfläche.

12. Verfahren zum Befestigen eines Lipid-umhüllten Partikels an einer Oberfläche, umfassend:
Bereitstellen eines Partikels in Nano- oder Mikrogröße, umfassend Membranproteine, die an der Oberfläche des Partikels immobilisiert sind, und einen Überzug aus einer Lipiddoppelschicht, der in die Räume zwischen den Membranproteinen eingedrungen ist und das Partikel umhüllt, wobei das Membranprotein an der Oberfläche des Partikels durch ein Linker-Molekül mit einer Länge von höchstens 5,5 nm in ausgestreckter Konfiguration immobilisiert ist, wobei das Partikel eine wässrige Schicht zwischen der Hülle aus der Lipiddoppelschicht und der Partikeloberfläche umfasst und wobei die Membranproteine innerhalb der Lipiddoppelschicht einheitlich orientiert sind mit Bezug zu der Partikeloberfläche, weiterhin umfassend ein an der Lipidschicht angeheftetes Ankermolekül oder Gruppe, das/die zur Anlagerung des Partikels an einer Oberfläche, vorzugsweise der Oberfläche einer Vertiefung einer Mikrotiterplatte, geeignet ist, wobei vorzugsweise das Partikel wie in einem der Ansprüche 2 bis 10 weiter definiert ist,
und Anlagern der Lipid-umhüllten Partikel an der Oberfläche.

13. Verfahren zum Testen einer biologischen Aktivität eines Membranproteins, umfassend das Bereitstellen eines Partikels gemäß einem der Ansprüche 1 bis 10 und Testen auf die biologische Aktivität eines Membranproteins innerhalb der Lipiddoppelschicht, die das Partikel umhüllt.

14. Verfahren zum Testen eines Membranproteins auf seine Fähigkeit, eine Kandidaten-Bindungssubstanz zu binden, umfassend das Bereitstellen eines Partikels gemäß einem der Ansprüche 1 bis 10, Hinzufügen einer Kandidaten-Bindungssubstanz und Bestimmen von Bindungsereignissen von der Kandidaten-Bindungssubstanz und den Membranproteinen in der Lipiddoppelschicht, die das Partikel umhüllt.

15. Verfahren gemäß Anspruch 14, wobei die Kandidaten-Bindungssubstanz eine aktive Kandidatensubstanz ist, die eine biologische Aktivität des Membranproteins potenziell modifiziert, wobei der Schritt des Bestimmens der Bindungsereignisse den Schritt des Bestimmens einer biologischen Aktivität von Interesse des Membranproteins umfasst.

16. Verfahren gemäß Anspruch 13 oder 15, wobei die biologische Aktivität ausgewählt ist aus einer enzymatischen Reaktion, dem Transport eines Moleküls oder Ions, vorzugsweise dem Transport eines Ions durch einen lonenkanal, und der Bindung eines Liganden.

17. Verfahren zur Herstellung einer haltbaren, rekonstituierbaren Präparation von Partikeln mit einer Lipiddoppelschicht, umfassend das Bereitstellen von Partikeln gemäß einem der Ansprüche 1 bis 10 und Einfrieren dieser Partikel, vorzugsweise Schockgefrieren dieser Partikel.

## Revendications

1. Particule de l'ordre du nanomètre ou du micromètre comprenant des protéines de membrane immobilisées à la surface de la particule et une feuille constituée d'une bicouche lipidique intercalée entre les protéines de membrane et enveloppant ladite particule, dans laquelle ladite protéine de membrane est immobilisée à la surface de la particule par une molécule de liaison ayant une longueur d'au plus 5,5 nm en configuration déployée, ladite particule comprenant une couche aqueuse entre l'enveloppe constituée de la bicouche lipidique et la surface des particules, et dans laquelle les protéines de membrane à l'intérieur de la bicouche lipidique sont systématiquement orientées par rapport à la surface des particules, comprenant en outre une molécule ou un groupe d'ancrage fixé(e) à la couche lipidique fixant la particule à une surface, de préférence une surface d'un puits de plaque de microtitration.

2. Particule selon la revendication 1, dans laquelle entre 10 % et 80 % de la surface de l'enveloppe lipidique sont constitués d'une protéine de membrane.

3. Particule selon la revendication 1 ou 2, dans laquelle la taille de la particule se situe dans une plage de 5 nm à 1 mm, de préférence de 30 nm à 900 µm, ou de 200 nm à 800 µm, de préférence de 800 nm à 600 µm, de manière particulièrement préférée de 1,5 µm à 500 µm, de manière encore davantage préférée de 10 µm à 200 µm.

4. Particule selon l'une quelconque des revendications 1 à 3, dans laquelle ledit lieur a une longueur d'au moins 0,5 nm, de préférence d'au moins 1 nm et/ou a une dimension latérale d'au plus 1 nm.

5. Particule selon l'une quelconque des revendications 1 à 4 dispersée dans un solvant aqueux.

6. Particule selon l'une quelconque des revendications 1 à 5, ladite particule étant constituée d'un matériau ou comprenant un matériau choisi parmi un polymère, de préférence un polymère de glucide, un métal ou un métalloïde, de préférence de l'Ag, de l'Au, du Si, du Ti, du Ta, du GeAs, ou une nanoparticule de carbone.

7. Particule selon l'une quelconque des revendications 1 à 6, dans laquelle une autre protéine de membrane, différente de ladite protéine de membrane immobilisée, est comprise dans ladite bicouche lipidique.

8. Particule selon l'une quelconque des revendications 1 à 7, dans laquelle ladite bicouche lipidique est une membrane de deux couches de molécules amphiphiles, de préférence dans laquelle lesdites molécules comprennent une partie hydrophobe d'une taille de C6 à C30, de préférence de C8 à C26.

9. Particule selon l'une quelconque des revendications 1 à 8, comprenant une autre protéine adhérant à ladite bicouche lipidique.

10. Particule selon l'une quelconque des revendications 1 à 9, dans laquelle la protéine de membrane à l'intérieur de la bicouche lipidique, immobilisée ou non immobilisée à la surface de la particule, ou la protéine adhérant à ladite bicouche lipidique est choisie parmi une intégrine, un canal ionique, une protéine transporteur, un récepteur membranaire, une protéine périphérique, une protéine associée à la membrane, une protéine redox.

11. Procédé de fabrication d'une particule comportant une enveloppe constituée d'une bicouche lipidique selon l'une quelconque des revendications 1 à 10, comprenant les étapes consistant à apporter une particule de l'ordre du nanomètre ou du micromètre, immobiliser les protéines de membrane à la surface de la particule, ajouter des molécules amphiphiles adéquates pour former une bicouche lipidique, pour ainsi obtenir des particules comportant ladite enveloppe constituée d'une bicouche lipidique, et fixer les particules à enveloppe lipidique comprenant une molécule ou un groupe d'ancrage, qui est fixé(e) à la bicouche lipidique, à une surface.

12. Procédé de fixation d'une particule à enveloppe lipidique à une surface comprenant
l'apport d'une particule de l'ordre du nanomètre ou du micromètre comprenant des protéines de membrane immobilisées à la surface de la particule et une feuille constituée d'une bicouche lipidique intercalée entre les protéines de membrane et enveloppant ladite particule, dans lequel ladite protéine de membrane est immobilisée à la surface de la particule par une molécule de liaison ayant une longueur d'au plus 5,5 nm en configuration déployée, ladite particule comprenant une couche aqueuse entre l'enveloppe constituée de la bicouche lipidique et la surface des particules, et dans lequel les protéines de membrane à l'intérieur de la bicouche lipidique sont systématiquement orientées par rapport à la surface des particules, comprenant en outre une molécule ou un groupe d'ancrage fixé(e) à la couche lipidique adapté(e) à la fixation de la particule à une surface, de préférence à la surface d'un puits de plaque de microtitration, de préférence ladite particule étant en outre définie comme dans l'une quelconque des revendications 2 à 10,
et la fixation des particules à enveloppe lipidique à la surface.

13. Procédé de détermination de l'activité biologique d'une protéine de membrane, comprenant l'apport d'une particule selon l'une quelconque des revendications 1 à 10 et la détermination de l'activité biologique d'une protéine de membrane à l'intérieur de la bicouche lipidique enveloppant la particule.

14. Procédé d'analyse d'une protéine de membrane pour déterminer sa capacité à se lier à une substance de liaison candidate, comprenant l'apport d'une particule selon l'une quelconque des revendications 1 à 10, l'ajout d'une substance de liaison candidate et la détermination des événements de liaison de ladite substance de liaison candidate et des protéines de membrane dans la bicouche lipidique enveloppant la particule.

15. Procédé selon la revendication 14, dans lequel ladite substance de liaison candidate est une substance active candidate modifiant potentiellement une activité biologique de ladite protéine de membrane, dans lequel l'étape de détermination des événements de liaison comprend l'étape de détermination d'une activité biologique d'intérêt de ladite protéine de membrane.

16. Procédé selon la revendication 13 ou 15, dans lequel ladite activité biologique est choisie parmi une réaction enzymatique, le transport d'une molécule ou d'un ion, de préférence le transport d'un ion par un canal ionique, et la liaison d'un ligand.

17. Procédé de préparation d'une préparation reconstituable durable de particules comportant une bicouche lipidique, comprenant l'apport de particules selon l'une quelconque des revendications 1 à 10 et la congélation desdites particules, de préférence la congélation choc desdites particules.
